# EUROPÄISCHE PATENTSCHRIFT

(11) **EP 0 542 129 B1**
(45) Veröffentlichungstag und Bekanntmachung des Hinweises auf die Patenterteilung: **14.06.1995**
(21) Anmeldenummer: 92118937.9
(22) Anmeldetag: 05.11.1992
(51) Int. Cl.: A61K 7/13

(54) **Haarfärbemittel**
Hair dye composition
Composition pour la teinture des cheveux

(30) Priorität: 11.11.1991 DE 4136997
(43) Veröffentlichungstag der Anmeldung: 19.05.1993
(73) Patentinhaber: GOLDWELL GmbH, D-64297 Darmstadt (DE)
(72) Erfinder: Lorenz, Heribert, W-6101 Gross-Bieberau (DE); Tennigkeit, Jürgen, Dr., W-6104 Seeheim-Jugenheim (DE); Kufner, Frank, W-6100 Darmstadt 13 (DE)

(56) Entgegenhaltungen:
- EP-A- 0 376 078
- DE-A- 2 629 805
- DE-A- 3 233 540
- DE-U- 9 105 827

## Beschreibung

Die vorliegende Erfindung betrifft ein neues Haarfärbemittel für menschliches Haar, das insbesondere verbesserte Farbeigenschaften und eine ausgezeichnete toxikologische und dermatologische Verträglichkeit aufweist.

Haarfärbemittel enthalten, wie in Fachkreisen allgemein bekannt, mindestens ein Oxidationsfarbstoff-Vorprodukt, eine sogenannte Entwicklerkomponente und Kuppler, die, nach Hinzufügung von Wasserstoffperoxid, unmittelbar vor der Haarfärbung zusammengebracht werden und dann die gewünschte Farbreaktion ergeben. Zur Feineinstellung des gewünschten Farbtones werden einer solchen Mischung üblicherweise auch Nuanceure zugesetzt. Übliche Entwicklersubstanzen sind insbesondere aromatische Diamine wie p-Phenylendiamin und p-Toluylendiamin. Diese Substanzen sind hinsichtlich ihrer dermatologischen Eigenschaften für manche Benutzer nicht optimal. Es besteht daher ein Bedürfnis, weitere Entwicklersubstanzen mit verbesserten dermatologischen und toxikologischen Eigenschaften zu finden.
In der eigenen DE-U 91 05 827.9 werden für diesen Zweck Haarfärbemittel vorgeschlagen, die als Entwicklersubstanz alleine oder in Mischung mit anderen Entwicklersubstanzen ein Hydroxytriaminopyrimidin und/oder ein Dihydroxidiaminopyrimidin enthalten, vorzugsweise 6-OH-2,4,5-triaminopyrimidin, 2-OH-4,5,6-triaminopyrimidin und 5-OH-2,4,6-triaminopyrimidin bzw. 2,6-Dihydroxy-4,5-diaminopyrimidin und/oder 4,6-Dihydroxy-2,5-diaminopyrimidin.

Als bevorzugte Kupplersubstanzen mit diesen neuen Entwicklersubstanzen werden dabei Resorcin, m-Aminophenol, m-Phenylendiamin, α-Naphthol, p-Amino-4-hydroxyethylaminoanisol, o-Aminophenol, o-Chlor-p-phenylendiamin, 1,7-Dihydroxynaphthalin und 3-Dimethylaminophenol eingesetzt, mit denen in der Regel gute Ausfärbungen erzielt werden, die jedoch den natürlichen Haarfarben nicht immer weitgehend entsprechen.

Die vorliegende Erfindung geht daher von der Aufgabenstellung aus, Kupplersubstanzen zu entwicklen, die in Kombination mit Triaminohydroxy- und Diaminodihydroxypyrimidin-Entwicklers eine möglichst natürliche Haarfärbung ergeben.

Die Lösung dieser Aufgabe besteht erfindungsgemäß darin, im Gemisch mit Triaminohyroxy- bzw. Diaminodihydroxypyrimidin als Entwickler enthaltenden Haarfärbemitteln als Kupplersubstanz 3-Amino-2-methylamino-6-methoxypyridin alleine oder im Gemisch mit anderen Kupplern, ausgenommen solchen der allgemeinen Formel 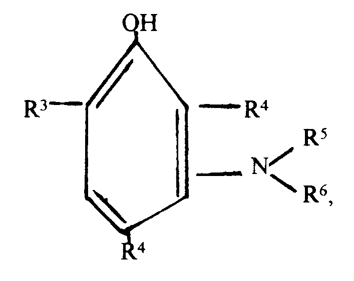
worin R³ H oder eine Methoxygruppe, R⁴, R⁵ und R⁶ H oder C₁-C₄-Alkylgruppen, oder R⁵ eine C₅-C₆-Cycloalkylgruppe bedeuten, zuzusetzen.
Durch die Kombination dieser speziellen Entwicklersubstanzen mit dem speziellen Kuppler werden, nach Zusatz von Wasserstoffperoxid oder anderen Peroxiden, Haarfärbungen mit einem exzellenten Naturfarbton erreicht.

Die Verwendung von 2,3-Diamono-6-methoxypyridin-Derivaten, beispielsweise 3-Amino-2-methylamino-6-methoxypyridin, als Oxidationsfarbstoff-Vorprodukte in Haarfärbemittel ist an sich bereits aus der DE-A 32 33 540 bekannt. Hier werden diese Stoffe jedoch ausschließlich in Kombination mit mindestens einem aromatischen Diamin, 2-Aminophenol und/oder 4-Aminophenol zur Erzeugung von insbesonders Blautönen eingesetzt.

Es war daher überraschend und nicht vorhersehbar, daß sich durch die erfindungsgemäße Kombination eines dieser Pyridinderivate, nämlich des 3-Amino-2-methylamino-6-methoxypyridins, mit einer völlig anderen, neuen Klasse von Entwicklern, nämlich Diaminohydroxy- bzw. Triaminohydroxypyrimidinen, natürliche Haarfärbungen erzielen lassen würden.

Bevorzugte Triaminohydroxypyrimidine bzw. Dihydroxydiaminopyrimidine, die im übrigen auch in Form ihrer Salze, beispielsweise als Hydrochlorid, eingesetzt werden können, sind die in der bereits erwähnten DE-U 91 05 827.9 genannten Verbindungen, d. h. beispielsweise 2,4,5-Triamino-6-hydroxypyrimidin, 4,5,6-Triamino-2-hydroxypyrimidin und 2,4,6-Triamino-5-hydroxypyrimidin sowie 2,6-Dihydroxy-4,5-diaminopyrimidin und 4,6-Dihydroxy-2,5-diaminopyrimidin.
Ihr Anteil in den erfindungsgemäßen Haarfärbemitteln beträgt zwischen etwa 0,05 und 5, vorzugsweise 0,1 und 4, insbesondere 0,5 und 3 Gew.-% der Gesamtzusammensetzung (ohne Oxidationsmittel).

Obwohl nicht erforderlich, können gegebenenfalls, falls zur Herstellung bestimmter Farbnuancen erwünscht, weitere an sich bekannte Entwicklersubstanzen wie p-Phenylendiamin, p-Toluylendiamin, 4-Aminophenol und Tetraaminopyrimidin bzw. dessen Derivate mitverwendet werden.

Die Kupplersubstanz, d. h. das 3-Amino-2-methylamino-6-methoxypyridin, liegt in den erfindungsgemäßen Haarfärbemitteln etwa im gleichen Anteil wie die Entwicklersubstanzen vor, d. h. also in Mengen von 0,05 bis 5,0, vorzugsweise 0,1 bis 4, insbesondere 0,5 bis 3 Gew.-% der Gesamtzusammensetzung (ohne Oxidationsmittel).

Auch hier ist es möglich, weitere bekannte Kupplersubstanzen mitzuverwenden, falls dies zur Erzielung bestimmter Farbnuancen erwünscht und erforderlich ist.
Die erfindungsgemäßen Zusammensetzungen können erwünschtenfalls auch sogenannte Nuanceure zur Feineinstellung des gewünschten Farbtones, insbesondere auch direktziehende Farbstoffe, enthalten. Solche Nuanceure sind beispielsweise Nitrofarbstoffe wie 2-Amino-4,6-dinitrophenol, 2-Amino-4-nitrophenol, 2-Amino-6-chlor-4-nitrophenol etc., vorzugsweise in Mengen von etwa 0,05 bis 2,5, insbesondere 0,1 bis 1 Gew.-% der Farbzusammensetzung (ohne Oxidationsmittel).

Die erfindungsgemäßen Haarfärbemittel können die in solchen Mitteln üblichen Grund- und Zusatzstoffe, Konditioniermittel, etc. enthalten, die dem Fachmann aus dem Stand der Technik bekannt und beispielsweise in der Monographie von K.Schrader, "Grundlagen und Rezepturen der Kosmetika", 2. Auflage (Hüthig Buch Verlag, Heidelberg, 1989), S. 782 bis 815, bekannt sind. Sie können als Lösungen, Cremes, Gele oder auch in Form von Aerosol-Zusammensetzungen vorliegen.

Zur Applikation wird das erfindungsgemäße Oxidationsfarbstoff-Vorprodukt mit einem Oxidationsmittel vermischt. Bevorzugtes Oxidationsmittel ist Wasserstoffperoxid, beispielsweise in 2- bis 6prozentiger Konzentration.
Es können jedoch auch andere Peroxide wie Harnstoffperoxid und Melaminperoxid eingesetzt werden.

Der pH-Wert des applikationsfertigen Haarfärbemittels, d. h. nach Vermischung mit Peroxid, kann sowohl im schwach sauren, d. h. einem pH-Bereich von 5,5 bis 6,9, im neutralen als auch im alkalischen Bereich, d. h. zwischen pH 7,1 und 9,5, liegen.

Eine besonders schonende Haarfärbung wird bei Applikation einer schwach sauren Färbemittelzusammensetzung erhalten, wobei durch die Verwendung des erfindungsgemäßen Kupplers auch besonders natürlich erscheinende Farbtöne erhalten werden.

Im folgenden werden verschiedene Ausführungsbeispiele zur Erläuterung der Erfindung gegeben.

### Beispiele

### A

In eine Cremegrundmasse der folgenden Zusammensetzung

| | |
|---|---|
| Cetylstearylalkohol | 12,00 (Gew.-%) |
| Cocosfettsäuremonoethanolamid | 2,00 |
| Stearinsäuremonoethanolamid | 2,00 |
| Ethoxyliertes (4 EO) Stearinsäuremonoethanolamid | 0,80 |
| Ölsäure | 1,20 |
| Natriumhydroxid | 0,25 |
| Natriumlaurylsulfat | 0,50 |
| EDTA | 0,20 |
| Ammoniumchlorid | 0,20 |
| Natriumsulfit | 0,25 |
| Eiweißhydrolysat | 1,00 |
| Mangandioxid | 0,12 |
| Parfum | 0,20 |
| Wasser | ad 100,00 |

wurden jeweils, unter entsprechender Reduktion des Wassergehaltes, folgende Oxidationsfarbstoff-Vorprodukte eingearbeitet:

| Nr. 1: | |
|---|---|
| 2,5,6-Triamino-4-hydroxypyrimidin | 1,66 (Gew.-%) |
| 3-Amino-6-methoxy-2-methylaminopyridin | 1,56 |
| 2,6-Diaminopyridin | 1,24 |

| Nr. 2: | |
|---|---|
| 2,5,6-Triamino-4-hydroxypyrimidin | 1,12 (Gew.-%) |
| 3-Amino-6-methoxy-2-methylaminopyridin | 1,04 |
| 2,6-Diaminopyridin | 0,14 |
| Picraminsäure | 0,04 |

| Nr. 3: | |
|---|---|
| 2,5,6-Triamino-4-hydroxypyrimidin | 0,90 (Gew.-%) |
| 3-Amino-6-methoxy-2-methylaminopyridin | 0,60 |
| Naphthalindiol-2,3 | 0,08 |
| 2-Methylresorcin | 0,16 |
| 2,5-Diaminopyridin | 0,16 |
| Picraminsäure | 0,04 |

| Nr. 4: | |
|---|---|
| 2,5,6-Triamino-4-hydroxypyrimidin | 0,80 (Gew.-%) |
| 3-Amino-6-methoxy-2-methylaminopyridin | 0,20 |
| 2,5-Diaminopyridin | 0,55 |
| 2-Methylresorcin | 0,10 |

| Nr. 5: | |
|---|---|
| 2,5,6-Triamino-4-hydroxypyrimidin | 0,80 (Gew.-%) |
| 3-Amino-6-methoxy-2-methylaminopyridin | 0,11 |
| 2,5-Diaminopyridin | 0,40 |
| 2-Methylresorcin | 0,30 |
| 2-Aminophenol | 0,10 |
| Picraminsäure | 0,05 |

| Nr. 6: | |
|---|---|
| 2,4-Dihydroxy-5,6-diaminopyrimidin | 0,76 (Gew.-%) |
| 3-Amino-6-methoxy-2-methylaminopyridin | 0,70 |

| Nr. 7: | |
|---|---|
| 4,6-Dihydroxy-2,5-diaminopyrimidin | 0,75 (Gew.-%) |
| 3-Amino-6-methoxy-2-methylaminopyridin | 0,70 |

| Nr. 8: | |
|---|---|
| 2-Hydroxy-4,5,6-triaminopyrimidin | 0,75 (Gew.-%) |
| 3-Amino-6-methoxy-2-methylaminopyridin | 0,70 |

Jeweils 20 g dieser Cremezusammensetzungen wurden mit 40 ml 2%iger H₂O₂-Lösung vermischt, wobei jeweils ein Produkt mit einem pH-Wert von 6,8 erhalten wurde, und auf menschliches Haar aufgebracht. Nach 15- bis 20-minütiger Einwirkung wurde ausgewaschen und gespült, wobei folgende natürliche Farbtöne erhalten wurden:
- Nr. 1:: Dunkelbraun
- Nr. 2:: Hellbraun
- Nr. 3:: Dunkelblond
- Nr. 4:: Rotbuche
- Nr. 5:: Mahagoni
- Nr. 6:: Mattes Goldblond
- Nr. 7:: Haselnuß
- Nr. 8:: Goldblond

### B

In eine Cremegrundmasse der Zusammensetzung

| | |
|---|---|
| Cetylstearylalkohol | 12,00 (Gew-%) |
| Cocosfettsäuremonoethanolamid | 2,30 |
| Stearinsäuremonoethanolamid | 2,30 |
| Oleylalkoholethoxylat (5 EO) | 0,50 |
| Ölsäure | 2,50 |
| 1,2-Propandiol | 2,00 |
| Natriumlaurylsulfat | 0,50 |
| EDTA | 0,20 |
| Ammoniumchlorid | 0,50 |
| Natriumsulfit | 0,50 |
| Ascorbinsäure | 0,50 |
| Eiweißhydrolysat | 1,00 |
| Ammoniak, 25 % | 7,20 |
| Parfum | 0,30 |
| Wasser | ad 100,00 |

wurden jeweils, unter entsprecheder Reduzierung des Wassergehaltes, folgende Oxidationsfarbstoff-Vorprodukte eingearbeitet:

| Nr. 1: | |
|---|---|
| 2,5,6-Triamino-4-hydroxypyrimidin | 1,40 (Gew.-%) |
| 3-Amino-6-methoxy-2-methylaminopyridin | 0,47 |
| 3-Amino-2-hydroxypyridin | 1,17 |
| Naphthalindiol-1,7 | 0,56 |

| Nr. 2: | |
|---|---|
| 2,5,6-Triamino-4-hydroxypyrimidin | 0,72 (Gew.-%) |
| 3-Amino-6-methoxy-2-methylaminopyridin | 0,65 |

| Nr. 3: | |
|---|---|
| 4,6-Dihydroxy-2,5-diaminopyrimidin | 0,75 (Gew.-%) |
| 3-Amino-6-methoxy-2-methylaminopyridin | 0,70 |

Jeweils 20 g dieser Cremezusammensetzungen wurden mit 20 ml 6%iger H₂O₂-Lösung vermischt, wobei ein Produkt mit einem pH-Wert von 9,5 erhalten wurde, und auf menschliches Haar aufgebracht. Nach 30-minütiger Einwirkung wurde gewaschen und gespült. Es wurden folgende Naturtöne erhalten:
- Nr. 1:: Kräftiges Rehbraun
- Nr. 2:: Kräftiges Aschblond
- Nr. 3: Dunkelmattblond

## Patentansprüche

1. Haarfärbemittel, das als Entwicklersubstanz mindestens ein Triaminohydroxypyrimidin und/oder Diaminodihydroxypyrimidin und zusätzlich Kupplersubstanz(en) enthält, dadurch gekennzeichnet, daß als Kupplersubstanz(en) 3-Amino-2-methylamino-6-methoxypyridin oder dessen wasserlösliche Salze alleine oder im Gemisch mit anderen Kupplersubstanzen, ausgenommen solchen der allgemeinen Formel 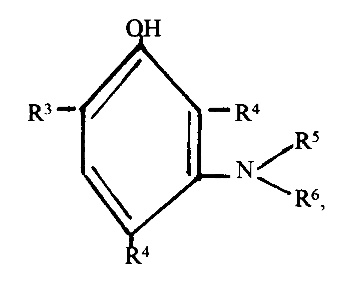 worin R³ H oder eine Methoxygruppe, R⁴, R⁵ und R⁶ H oder C₁-C₄-Alkylgruppen, oder R⁵ eine C₅-C₆-Cycloalkygruppe bedeuten, eingesetzt werden.

2. Haarfärbemittel nach Anspruch 1, dadurch gekennzeichnet, daß es das 3-Amino-2-methylamino-6-methoxypyridin in einer Menge zwischen 0,1 und 3 Gew.-%, bezogen auf die Gesamtzusammensetzung, enthält.

3. Haarfärbemittel nach Anspruch 1 oder 2, dadurch gekennzeichnet, daß es nach dem Vermischen mit Peroxid in der gebrauchsfertigen Zusammensetzung einen pH-Wert zwischen 5,5 und 6,9 aufweist.

## Claims

1. Hair dye composition, containing as developing substances at least one triaminohydroxypyrimidine and (or) diaminodihydroxypyrimidine and additional coupling agent(s), characterized in that it contains 3-amino-2-methylamino-6-methoxy-pyridine or the water-soluble salts thereof as coupling agent alone or in combination with additional coupling substances, except those corresponding to the general formula 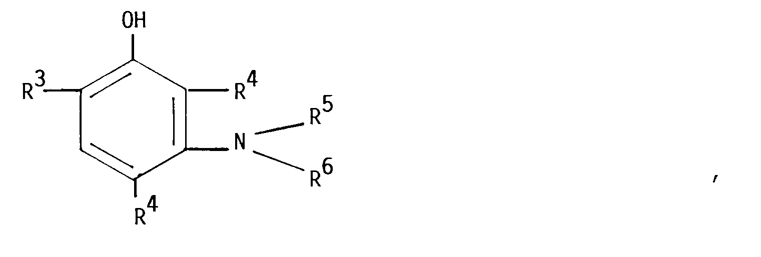 wherein R³ is H or a methoxy group, and R⁴, R⁵, and R⁶ are H or C₁-C₄-alkyl groups, or R⁵ is a C₅-C₆-cycloalkyl group.

2. Hair dye composition according to claim 1, characterized in that it contains 3-amino-2-methylamino-6-methoxypyridine in an amount of 0.1 to 3% by weight, calculated to the total composition.

3. Hair dye composition according to claim 1 or 2, characterized in that, after mixing with peroxide, the ready-to-use preparation has a pH-value between 5.5 and 6.9.

## Revendications

1. Composition pour la teinture des cheveux, qui contient comme substance révélatrice au moins une triamino-hydroxypyrimidine et/ou une diaminohydroxypyrimidine et en outre une (des) substance(s) de couplage, caractérisée en ce que l'on utilise comme substance(s) de couplage la 3-amino-2-méthylamino-6-méthoxypyridine ou ses sels hydrosolubles, seuls ou en mélange avec d'autres substances de couplage, à l'exception de celles qui répondent à la formule générale 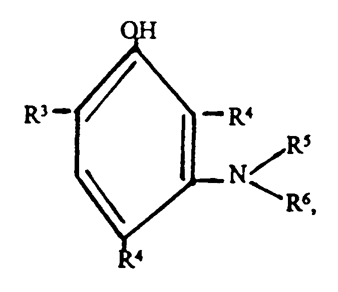 dans laquelle R³ représente H ou un groupe méthoxy, R⁴, R⁵ et R⁶ représentent H ou des groupes alkyles en C₁-C₄, ou R⁵ représente un groupe cycloalkyle en C₅-C₆.

2. Composition pour la teinture des cheveux selon la revendication 1, caractérisée en ce qu'elle contient la 3-amino-2-méthylamino-6-méthoxypyridine en une quantité entre 0,1 et 3 % en poids, rapporté à la composition totale.

3. Composition pour la teinture des cheveux selon l'une des revendications 1 ou 2, caractérisée en ce qu'elle présente, dans la composition prête à l'emploi après mélange avec un peroxyde, une valeur de pH entre 5,5 et 6,9.
